# EUROPEAN PATENT APPLICATION

(11) **EP 4 006 173 A1**
(43) Date of publication of application: **01.06.2022**
(21) Application number: 21218171.3
(22) Date of filing: 10.12.2019
(51) Int. Cl.: C12R 1/00, A01C 1/06, A01N 63/20, C05F 11/08, C12R 1/41

(54) **MICROBIAL COMPOSITIONS BASED ON NATIVE RHIZOBIA STRAINS AND METHOD OF IMPROVING LEGUME PASTURES IN ACID SOILS WITH MANGANESE TOXICITY**

(62) Divisional of application: 19215059.7
(71) Applicant: Universidade de Évora, 7000-803 Évora (PT)
(72) Inventor: Carvalho, Mário, 7000-712 Évora (PT); Alho, Luís, 7000-171 Évora (PT); Alexandre, Ana, 7005-546 Évora (PT); Brígido, Clarisse, 7005-260 Évora (PT); Menendez, Esther, 7000-573 Évora (PT)
(74) Representative: Gata-Goncalves, Ligia

(57) **Abstract**

The present invention refers to microbial compositions based on native rhizobia strains belonging to different species of the genera *Rhizobium* and *Ensifer,* to these microbial strains and to methodologies for improving pastures and forages through the application of these compositions.

These compositions are used to improve the productivity of pastures by improving the growth of plants, namely legumes, in particular clovers and lucerne, which are widely used in acidic soils with Manganese toxicity problems in the "Montado" system.

Thus, the present invention has application in the field of agriculture, especially in sustainable agriculture, as a way to potentiate the growth of plants, particularly in situations of abiotic stress.

## Description

### TECHNICAL DOMAIN OF THE INVENTION

The present invention refers to microbial compositions based on native rhizobia strains belonging to different species of the genera *Rhizobium* and *Ensifer,* to these microbial strains and to methodologies for improving pastures and forages through the application of these compositions.

The microbial compositions of the present invention comprise at least one of four rhizobia strains, isolated and selected from leguminous plants grown in acidic soils, belonging to the genera *Rhizobium* and *Ensifer.*

In another aspect, the present invention relates to a method of improving pastures and forages, with legumes and/or a combination of legumes with non-legume plants, for example grasses, by applying the compositions of the invention as a plant inoculum, with the aim of improving the growth and quality of pastures and reducing or eliminating the use of chemical treatments that often have negative consequences for the environment and health.

Thus, the present invention has application in the field of agriculture, microbiology and phyto-pharmacology, especially in sustainable agriculture, as a way to potentiate the growth of plants, particularly in situations of abiotic stress.

### BACKGROUND OF THE INVENTION

**"Montado"** is a particular agro-silvo-pastoral ecosystem, typically composed of holm oaks, cork oaks, oaks and/or chestnut trees, with a very delicate balance and which only subsists under Mediterranean climate conditions, namely in Algeria, Morocco and especially in South of the Iberian Peninsula. These systems are often exploited as a forest and aim at establishing cattle pastures.

In Portugal, the country with the largest area of cork oak forest in the world, which corresponds to around 33% of the world area, the "Montado" has been legally protected since 2001, its felling has been prohibited, and its exploration is encouraged. Portugal is also the leading world exporter of cork, which shows the importance of "Montado" also in economic terms. This ecosystem also exists in Spain, where it is known as *dehesa.*

Soil acidity restricts crop production to about 70% of the world's potentially arable soils (85% in Portugal), mainly due to the effects associated with manganese (Mn) and aluminum (Al) toxicity on plant growth. Portuguese soils occupied by the "Montado" system are mostly acid and have low fertility, which compromises the animal production as well as the productivity of the system's own tree cover.

Animal feed in this system is generally based on natural pastures, typically not very productive, or on sown pastures composed by leguminous plants, namely annual clovers and lucerne, and also fine grains for hay.

However, due to the frequent problems of acidity in these soils, the success of sown pastures is reduced, and the persistence of sown plant species is often not superior to two years. In addition to the direct effect of acidity, the effect of manganese toxicity on plants is complex, and it is not possible to define an evident threshold for toxicity its concentration, mainly because its harmful effects depend on the relative concentration of other ions and other compounds, such as indole acetic acid (IAA), amongst others.

Manganese is found in the soil mainly in the form of oxide, but also in the form of silicates. Mn²⁺ ions are released into the soil solution during the silicate weathering. As the clay content of the soil, the pH and the redox potential of the soil are important factors in determining the potential of a soil and in the retention of this exchangeable manganese. By lowering the pH value and the redox potential, the concentration of manganese ions available in the soil and, consequently, in the plant increases. Manganese is absorbed by the plant only as an Mn²⁺ ion. This process can be inhibited by high concentrations of Mg²⁺, Ca²⁺, Cu²⁺ ions and also iron. Manganese is an essential component of many enzymes and therefore strongly influences the metabolism of the plant.

In addition to their effect on plant performance, excessive concentrations of manganese in the soil affect the growth and performance of soil microbes. In the case of legumes, such as the subterranean clover, the plant's tolerance to the toxic effect of manganese appears to be greater than that of the associated rhizobia, with the symbiotic system and consequently the nitrogen fixation being severely affected by toxicity. Furthermore, the success of rhizobia inoculation under these conditions has been limited due to the reduced performance of traditional inoculants under acidic and soil toxicity conditions present in the soils of the "Montado" system.

It is therefore essential to develop rhizobia inoculants adapted to the most prevalent soil conditions in the "Montado" system in order to improve the productivity of pastures with legumes.

The present invention proposes as a solution to this problem, microbial compositions, based on specific rhizobia strains and selected according to their characteristics, and their use as an inoculum in pastures with legumes, especially under conditions of acidity and associated manganese toxicity (Mn). These strains have a high tolerance to stress conditions and a higher efficiency in fixing N₂, as well as a highly competitive ability, having been isolated from the native populations of "Montado" soils.

### GENERAL DESCRIPTION OF THE INVENTION

The present invention refers to microbial compositions based on at least one of the four rhizobia strains T1, T2, M1 and M2 of rhizobia, belonging to the Family Rhizobiaceae, namely to species within the genera *Rhizobium* and *Ensifer,* to these microbial strains and to a method for improving of pastures and forages, with legumes and/or in association with other types of plants, by applying the microbial compositions of the invention to these plants, in particular in the ones grown in acidic soils with toxicity problems, namely those plants that are grown in the "Montado" system, with the aim of improving the growth and quality of these pastures and reducing or eliminating the use of chemical treatments that often have negative consequences for the environment and health.

### 1. Obtention and isolation of microbial strains

The obtention and isolation of the strains of the microbial compositions of the present invention is done, in a first stage, through the collection of roots of leguminous plants and, then, by the selection of the best rhizobia root nodules, with the objective of being used as a plant inoculum, and having good symbiotic efficiency (SE) between the microorganism and the host plant.

In the context of the present invention, **Symbiotic Efficiency** (SE) is the percentage expression of the ratio between the dry matter produced by plants inoculated with rhizobia and the dry matter produced by uninoculated plants but supplemented with mineral nitrogen, in the amount necessary to fully satisfy the nitrogen needs of the plant, as described by Gibson AH "Evaluation of nitrogen fixation by legumes in the greenhouse and growth chamber. In: Elkan GH (ed) Symbiotic nitrogen fixation technology. Marcel Dekker, Inc, New York, pp 321-363, 1987).

In the context of the present invention, the term "manganese stress" means that plants are exposed to an excess of this element, which is eventually considered sufficient to induce "manganese toxicity".

For this purpose, roots are collected from leguminous plants, such as clover and lucerne, naturally infected by rhizobia, preferably being collected from different types of acidic soil and with manganese toxicity.

The nodules are selected according to their growth and development, especially the prominent nodules inserted in the main root with a red coloration, which is an indicator of the presence of leghemoglobin, therefore, directly related to the N₂ fixation activity in the nodule. These characteristics are the most promising ones for the successful isolation of effective rhizobia.

The selected nodules are processed, for example by crushing and macerating, for a better exposure of the microorganisms, and grown in a suitable culture medium, such as Mannitol Yeast Agar (MLA) supplemented with Congo Red and cycloheximide, for 5 days at 28°C.

Typically, the MLA medium is composed by K₂HPO₄ in a concentration of 0.675 g/L, MgSO₄ in a concentration of 0.2 g/L, NaCl at 0.1 g/L, mannitol at 10 g/L, Yeast extract at 0.5 g/L and Agar at 15 g/L. The concentration of Congo Red and cycloheximide is 0.0025 g/L and 0.004 g/L, respectively.

### 2. Selection of microbial strains

The selection of microbial strains is carried out from a collection of pure cultures, obtained as described above, taking into account the following 4 criteria:
**A. Efficiency in N₂ fixation under manganese stress conditions,** assessed through the number of N₂-fixing nodules, the Symbiotic Efficiency (SE) and the growth of plants subject to manganese stress;
**B. Efficiency in N₂ fixation without manganese stress conditions,** assessed through the parameters described above, but for plants not subject to manganese stress;
**C. Saprophytic tolerance at 30 ppm Mn,** determines the survival and resistance ability of the microbial strains of the invention in relation to manganese stress and can be assessed by determining the growth of the strains in liquid medium supplemented with different amounts of Mn in parts per million (ppm), namely 30ppm of MnSO₄.
**D. Ability to produce indole acetic acid (IAA):**
   Since the production of indole acetic acid (IAA) is a recognized factor in promoting plant growth, particularly when plants are subjected to stress conditions, strains were preferably selected according to their IAA production ability.

The assessment of the strains' ability to produce indole acetic acid (IAA) is carried out as follows: inoculation of the bacterial culture in minimal liquid M9 medium, as described by Duan et al. 2009 (Duan, J., Muller, K.M., Charles, T.C. et al. Microb Ecol (2009) 57: 423. https://doi.org/10.1007/s00248-008-9407-6) and supplemented with tryptophan. The cultures are incubated at 28-30°C until they reach the stationary growth phase (48-72 h).

Salkowski's reagent is added to the supernatant of these cultures, as described by Gordon & Weber 1951 (Gordon SA and Weber R P 1951. Plant Physiol. 26, 192-195.), followed by an incubation in the dark for about 20-30 min at room temperature (20-25°C).

After this incubation, the absorbance of each sample is measured at 535 nm and the IAA concentration is determined by comparing the values obtained with those of a standard curve, previously obtained with known concentrations of IAA ranging from 0.01 to 100 µg/mL IAA.

### E. Other factors contributing to promote plant growth and polysaccharide production ability

In order to assess other plant growth promoting capabilities of the selected strains, their ability to solubilize phosphorus and potassium and their ability to synthesize siderophores, i.e. compounds that capture iron, were determined.

The assessment of the strains' ability to produce siderophores is carried out by inoculating the microbial cultures in a solid medium designated M9-CAS-Agar, as described by Alexander & Zuberer in "Alexander, D.B., & Zuberer, D.A. (1991). Use of chrome azurol S reagents to assess siderophore production by rhizosphere bacteria. Biology and Fertility of Soils, 12(1), 39-45. doi:10.1007/bf00369386)". After incubation, the presence of an orange halo around the microbial cultures is indicative of the ability of these strains to produce siderophores. The absence of the halo is considered as the absence of such ability.

The assessment of the phosphorus and potassium solubilization ability is performed through the inoculation of microbial cultures in two solid media designated YED-P, as described by Peix in "Peix, A., et al. - Growth promotion of chickpea and barley by a phosphate solubilizing strain of Mesorhizobium mediterraneum under growth chamber conditions. Soil Biology and Biochemistry 33.1 (2001): 103-110)", and by Aleksandrov, as described by Sheng, 2005 (Sheng, X.F. (2005). Growth promotion and increased potassium uptake of cotton and rape by a potassium releasing strain of Bacillus edaphicus. Soil Biology and Biochemistry,37, 10:1918-1922, doi:10.1016/j.soilbio.2005.02.026.), respectively. After incubation, the presence of light halos around the microbial cultures is considered as a positive result. The absence of any halo is considered as absence of phosphorus or potassium solubilization ability.

In order to predict whether any strain will succeed in the adhesion and colonization of the host plant, its ability to produce polysaccharides, namely cellulose or similar molecules having a β(1-4)-glucan, was assessed.

The assessment of the strains' ability to produce these polysaccharides is determined by inoculating the microbial cultures in a solid MLA medium supplemented with 25mg/mL Congo Red, as described by Robledo et al. 2012 (Robledo, M., Rivera, L., Jiménez-Zurdo, J.I. et al. Role of Rhizobium endoglucanase CelC2 in cellulose biosynthesis and biofilm formation on plant roots and abiotic surfaces. Microb Cell Fact 11, 125 (2012) doi:10.1186/1475-2859-11-125). After incubation, colonies with said ability develop a reddish-colored mucous polysaccharide and are therefore considered positive for the test.

### 3. Microbial strains

Several strains were collected, cultivated and isolated, which were subsequently tested and selected taking into account the weighted criteria described above (A, B, C and D), as well as the intended use for each composition, according to its most relevant characteristics.

In particular, the following strains of microorganisms were selected: **T1, T2, M1 and M2,** the respective taxonomic classification being as follows:
**Microorganisms T1 and T2:** Kingdom: Bacteria, Phylum: Proteobacteria, Order: Rhizobiales, Family: Rhizobiaceae, Genus: Rhizobium, Species: *Rhizobium leguminosarum* sv *trifolii.*
**Microorganisms M1 and M2:** Kingdom: Bacteria, Phylum: Proteobacteria, Order: Rhizobiales, Family: Rhizobiaceae, Genus: *Ensifer,* Species: *Ensifer medicae.*

The microorganisms present in the microbial compositions were deposited, on 13 June 2019, in the "Colección Española de Cultivos Tipo" (CECT), International Depositary Authority (IDA) recognized under the Budapest Treaty for depositing microorganisms in patent-related procedures, whereupon they were given the following (i) name, (ii) identification given by the depositor (Universidade de Évora), and (iii) accession number assigned by the depositary (CECT):
- **T1:** (i) *Rhizobium leguminosarum,* (ii) TTR4, and (iii) CECT 9929;
- **T2:** (i)*Rhizobium leguminosarum,* (ii) RTR25, and (iii) CECT 9930;
- **M1:** (i) *Ensifer medicae,* (ii) RMP15, and (iii) CECT 9931;
- **M2:** (i) *Ensifer medicae,* (ii) MMP28, and (iii) CECT 9932.

According to the criteria used to select the strains, the native strains indicated above have high symbiotic efficiency (SE) in legume species grown in acidic soils, namely those existing in the "Montado" system. In addition, they present mechanisms related to plant growth promotion, namely the production of auxins, such as the indole acetic acid and indole acetic acid -like molecules, which can benefit different plant species, namely grasses, legumes and other non-legume plants.

**Table 1** below shows the isolated and selected strains, according to the present invention, as well as their main characteristics, it should be noted that clovers and lucerne are original hosts, respectively of the rhizobia T1 and T2, and M1 and M2.

**Table 1. Microbial strains and most relevant characteristics**

| **ID** | **ACCESS NO** | **SPECIES** | **CHARACTERISTICS** |
|---|---|---|---|
| **T1** | CECT 9929 | *R. leguminosarum* | N₂-fixing symbiont of clover N₂ fixation. Saprophytic tolerance at 30ppm Mn; N₂ fixation WITHOUT/WITH Mn stress; IAA production. Weak in cellulose synthesis. |
| **T2** | CECT 9930 | *R. leguminosarum* | N₂-fixing symbiont of clover N₂ fixation. Saprophytic tolerance at 30ppm Mn; N₂ fixation WITHOUT/WITH Mn stress; IAA production. Synthesizes cellulose and siderophores. |
| **M1** | CECT 9931 | *E.medicae* | N₂-fixing symbiont of lucerne N₂ fixation. Saprophytic tolerance at 30ppm Mn; N₂ fixation WITHOUT/WITH Mn stress; IAA production. Solubilizes P and K. Synthesizes cellulose. |
| **M2** | CECT 9932 | *E. medicae* | N₂-fixing symbiont of lucerne N₂ fixation. Saprophytic tolerance at 30ppm Mn; N₂ fixation WITHOUT/WITH Mn stress; IAA production. Weak in K solubilization. |

Thus, in another aspect, the present invention relates to these four isolated microbial strains, which are useful for the production of compositions based on a consortium of rhizobia with application in agriculture, in methodologies for improving pastures and forages.

### 4. Microbial Compositions

The microbial compositions according to the present invention comprise a consortium or a combination of the 4 microbial strains described above, namely strains T1, T2, M1 and M2. The weighting used favors the phenotypic characteristics complementarily, namely the nitrogen fixing ability and tolerance to high Mn concentrations, presented by each strain.

The Base Microbial Compositions (BMCs) of the invention comprise at least one of the four microbial strains, selected from T1, T2, M1 and M2, as described above.

In one embodiment of the invention, BMCs comprise more than one of the microbial strains of the invention, more preferably, the microbial compositions comprise mixtures of *Rhizobium* and *Ensifer* strains.

In another embodiment of the invention, the amounts and ratios of each of the microbial strains present in the BMCs are variable.

In another aspect of the present invention, BMCs comprise at least one rhizobia strain (T1 and/or T2) and at least one *Ensifer* strain (M1 and/or M2), in varying amounts, wherein the *Rhizobium:Ensifer* ratio ranges from 50:50%, 25:75%, 75:25%, 30:70%, 70:30%, 40:60%, 60:40%.

In another aspect of the present invention, BMCs comprise the four microbial strains (T1, T2, M1 and M2), in varying amounts, such as compositions **BMC-01** to **BMC-06** below:

| | **% T1** | **% T2** | **% M1** | **% M2** |
|---|---|---|---|---|
| **BMC-01** | 25.0 | 25.0 | 25.0 | 25.0 |
| **BMC-02** | 45.0 | 5.0 | 45.0 | 5.0 |
| **BMC-03** | 5.0 | 45.0 | 5.0 | 45.0 |
| **BMC-04** | 37.5 | 12.5 | 37.5 | 12.5 |
| **BMC-05** | 12.5 | 37.5 | 12.5 | 37.5 |
| **BMC-06** | 40.0 | 40.0 | 10.0 | 10.0 |

The presence of different relative amounts of each of the microbial strains, according to the above, allows obtaining different formulations of microbial compositions suitable for the application to different types of soil and pasture, depending on their most relevant characteristics (A, B, C and D) and, therefore, modeling each microbial composition for each type of intended use.

The effective number of bacteria in the microbial compositions is at least 10³ colony forming units per mL of composition (cfu/mL). This amount can be assessed taking into account the linear relationship between the absorbance or optical density (O.D.) of the culture and the total number of cells or cfu per milliliter of suspension.

However, the microbial compositions comprise a microbial charge greater than 10³ cfu/mL, preferably from 10⁶ to 10¹⁶ cfu/mL and from 10¹⁰ to 10¹² cfu/mL of the total composition and finally, more preferably from 10⁸ to 10¹⁴ cfu/mL.

Thus, in order to be able to guarantee that the number of cfu/mL is at least 10⁸ cfu/mL, in the microbial composition, it must have an O.D. of 0.5.

Thus, the microbial compositions of the present invention can be obtained by mixing at least one microbial isolate from one of the strains selected for the purpose, to a buffered saline solution, in which the total final microbial concentration must be greater than 10³ cfu/mL, preferably ranging from about 10⁶ to 10¹⁶ cfu/mL, more preferably from 10⁸ to 10¹⁴ cfu/mL, preferably from 10¹⁰ to 10¹² cfu/mL of the total final composition.

Typically, a buffered saline solution suitable for this type of suspension comprises, for example, a 0.85% NaCl solution to maintain osmotic balance and ensure that bacterial cells survive.

Although clovers are the original hosts of rhizobial strains T1 and T2, and lucerne the hosts of rhizobial strains M1 and M2, it is possible to observe that the effect of cross-inoculation, i.e. the effect of T1 and T2, when inoculated in lucerne, and M1 and M2 when inoculated in clover, and do not lead by themselves, to the formation of nitrogen fixing nodules in those non-host plants, i.e. T1 and T2 in lucerne and M1 and M2 in clover, but they can have other plant growth promotion effects and contribute to tolerance to Mn.

Thus, the microbial compositions of the invention, according to the above, show superior symbiotic efficiency in legume cultures used in pastures in the "Montado" system and also show other biological activities related to the promotion of plant growth.

### 5. Microbial formulations

Other compositions, derived from the Base Microbial Compositions (BMC) described above, can be produced by the addition of adjuvant compounds, dispersants, stabilizing agents, pH correction agents, vitamins, growth factors, etc., whose function is to improve the performance of pastures and forages where BMCs are applied, including plant protection and fertilizer formulations.

Thus, in another aspect, the present invention discloses Microbial Formulations comprising the Base Microbial Compositions and the agents and/or compounds described above.

Advantageously, these Formulations may comprise compounds, such as: black peat, white peat, alginate and other polysaccharides, sand, seeds, solutions, such as saline solutions or containing P or K, Ca, CaO, etc., as carriers that facilitate the application of microbial formulations to pastures and forages.

### 6. Method of improving pastures and forages

The application of the microbial compositions and formulations of the present invention can be carried out by any of the usual methods of applying inocula, for example, liquid application to the seed (immersion of the seed in the suspension of cells in buffer or saline), liquid application to the soil (using a suspension of cells in buffer or saline) or pelletizing seeds (adherence of cells to the seed) with vehicle for the inoculum (usually peat).

The compositions and formulations of the present invention are preferably applied to plants in the form of a suspension of microbial cells and can also be applied in carriers such as alginate, peat, among others.

Preferably, the compositions and formulations of the invention should provide a minimum population of 10³ cfu/seed, in order to guarantee the survival conditions of the strains.

Pastures and forages based on leguminous plants, or combinations of plants that include legumes, benefit from the application of the basic microbial composition as described above, especially when established in acidic soils and/or with problems of toxicity associated with manganese (Mn), as is the case of "Montados".

Leguminous forage plants include lupins and yellow lupins (*Lupinus sp.* including *L. albus, L. luteus, L. angustifolius*), vetches (*Vicia sp.,* including *V. sativa, V. faba, V. villosa, V. ervília, V articulata, V. narboensis, V. benghalensis, V. pannonica*) and sawdust (*Ornithopus sativus sp.*).

Forage legumes, particularly relevant to the "Montado" system include clovers (*Trifolium incarnatum, T. suaveolens, T. michelianum, T. resupinatum, T. vesiculosum, T. subterraneum* ssp *brachycalicinum, T. subterraneum* ssp *yanninicum, T. subterraneum ssp subterraneum, T. pratense, T campestre, T. tomentosum, T. angustifolium, T. fragiferum*) and lucerne (*Medicago polymorpha, M. truncatula, M. arabica, M. scutellata, M. murex*).

The application of the compositions and formulations of the invention is preferably carried out during the planting season of the plants of interest. However, this application can still be carried out after the seed emerges. Repeating the application of microbial compositions and formulations is also foreseen, within the scope of the invention, especially in cases of highly acidic soils, with little aeration, or with other problems associated with acidity or toxicity by metals, such as excess of manganese.

Thus, in a preferential aspect it is recommended to apply the microbial composition when sowing legumes, as well as re-application after the seed germination.

### EXAMPLES

### Example 1. Isolation of microbial strains

Root nodules selected according to the general description of the invention from the roots of clover and lucerne plants grown in acidic soils and with excess of Mn, were crushed in a sterile isotonic solution (8.5 g/L NaCl in distilled water), and the resulting macerates were inoculated in Petri dishes containing Mannitol and Yeast Agar (MLA) medium supplemented with Congo Red and cycloheximide.

### Composition of the MLA medium:

K₂HPO₄ = 0.675 g/L,
MgSO₄ = 0.2 g/L,
NaCl = 0.1 g/L,
Manitol = 10 g/L,
Yeast Extract = 0.5 g/L,
Agar = 15 g/L,
Congo Red = 0.0025 g/L, and
Cycloheximide = 0.004 g/L.

The cultures were incubated for 5 days at 28 ° C, and then, 270 colonies were selected to obtain pure cultures, which were subcultured in MLA medium without Congo Red.

### Example 2. Inoculation of microbial strains and plant growth IN manganese stress conditions (+MN)

The four strains selected from pure rhizobial cultures, **T1, T2, M1 and M2,** obtained as described in Example 1, were individually inoculated in plants of *Trifolium subterraneum* (subterranean clover) and plants of *Medicago polymorpha* (lucerne) subject to manganese stress.

For this purpose, 100 clover seeds and 200 lucerne seeds were disinfected on the surface, for 30 minutes, in 70% Ethanol + 4 min 0.1% HgCl₂ + 6 washes in sterile distilled water and germinated in water agar (0.75%) for 2 days. After germination, the resulting seedlings were placed in 8.5 x 8.5 cm pots (2 seed of clover and 2 of lucerne per pot), with a mixture of vermiculite:sand (1:1), which was led to saturation with a 0.5 mM solution of MnSO₄, in order to induce manganese stress. The seedlings were subject to acclimatization in a chamber and then individually inoculated with 1 mL of a bacterial solution with an optical density at 600nm (OD₆₀₀) of 0.5 (10⁸ cfu/mL).

The plants were kept under controlled conditions and watered, with a nutrient solution supplemented with manganese (0.5 mM MnSO₄) whenever the field capacity decreased by 5%, for 6 weeks.

The irrigation solution, suitable for the cultivation of clover and lucerne plants, was composed by macro- and micronutrients, with the exception of nitrogen, and resulted from the mixture of 4 solutions (S1 to S4), with plant nutrition functions. The nutrient solution contains all nutrients that the plant needs, with the exception of nitrogen, which is given by symbiosis with rhizobia, according to the formulations below:

### Composition of the nutrient solution:

**S1:** CaCl₂ at 1.0 mM;
**S2:** KH₂PO₄ at 0.5 mM;
**S3:** FeC₆H₅O₇ at 10 µM, MgSO₄ at 0,25 mM, K₂SO₄ at 0.25 mM and MnSO₄ at 1 µM; and
**S4:** H₃BO₃ at 2 µM, ZnSO₄ at 0.5 µM, CuSO₄ at 0.2 µM, CoCl₂ at 0.1 µM and Na₂MoO₄ at 0.1 µM.

To the nutrient solution described above, a 0.5 mM MnSO₄ solution was added to simulate the toxicity present under natural conditions of the soils.

After approximately 6 weeks, the plants were harvested, being entirely extracted from the pot and the substrate was removed, their growth and the number of N₂-fixing nodules were assessed, and the Symbiotic Efficiency (SE) was estimated. For the calculation of symbiotic efficiency (SE), the Gibson's formula (1987) was applied. The results are shown in table 2.

### Example 3. Inoculation of microbial strains and plant growth WITHOUT manganese stress conditions (-Mn)

Strains **T1, T2, M1 and M2** were individually inoculated into germinated seeds/seedlings of *Trifolium subterraneum* (subterranean clover) and *Medicago polymorpha* (lucerne), as described in the previous example. The plants were not subject to manganese stress, i.e., they were not subject to saturation with a 0.5 mM MnSO₄ solution.

The plants were kept in controlled conditions and watered, with an irrigation solution, as the one described in the previous Example without manganese supplement, whenever the field capacity dropped to 5%, for 6 weeks. The plants growth and the number of N₂-fixing nodules were assessed, and the Symbiotic Efficiency (SE) was estimated. The results are shown in table 2.

### Example 4. Effect of Mn stress on clover and lucerne plants

Clover and lucerne plants were inoculated with each of the rhizobia strains **T1, T2, M1 and M2** of the present invention, and grown under manganese stress (+Mn) conditions or without manganese stress (-Mn) for 6 weeks, as described in Examples 2 and 3.

As previously mentioned, after approximately 6 weeks, the plants were harvested, being their growth and the number of N₂-fixing nodules assessed and the Symbiotic Efficiency (SE) estimated. The comparative results of the SE and the total number of rhizobia-induced root nodules related to plants inoculated with the strains of the invention and grown under (+Mn) or (-Mn) conditions are shown in Table 2.

**Table 2. Symbiotic efficiency and number of nodules resulting from the individual inoculation of each selected strain in subterranean clover and lucerne plants (n=12 for each host plant, being n the number of individuals).**

| | **WITHOUT Mn stress (-Mn)** | | **WITH Mn stress (+Mn)** | |
|---|---|---|---|---|
| **Strain** | **SE (%)** | **No. of nodules** | **SE (%)** | **No. of nodules** |
| **SUBTERRANEAN CLOVER** | | | | |
| **T1** | 60.42 | 289 | 45.04 | 347 |
| **T2** | 44.15 | 732 | 82.35 | 806 |

| **LUCERNE** | | | | |
|---|---|---|---|---|
| **M1** | 60.20 | 216 | 98.09 | 189 |
| **M2** | 39.78 | 164 | 253.72 | 221 |

T1 and T2 were inoculated only in Clover while M1 and M2 were exclusively inoculated in lucerne, since these combinations are specific N₂-fixing microsymbionts of these plants. Although possible beneficial effects of these strains on non-specific hosts may exist, they are not due to the ability to fix atmospheric N₂.

The results obtained show how the strains improve the SE and NN of the plants under manganese stress conditions, except for strain T1 which only improves the total number of nodules. These data indicate that the rhizobia-legume symbiotic relationship is not negatively affected by the presence of manganese and that the symbiotic performance of these strains is improved under these conditions.

### Example 5. Measurement of saprophytic tolerance of microbial strains at 30ppm manganese

Strains T1, T2, M1 and M2 were incubated at 28 °C for 3 days in minimal medium (MM) supplemented with different MnSO₄ concentrations, ranging from 0 mM to 5 mM MnSO₄, in order to induce stress to manganese, according to the methodology described by Brígido *et al.* 2017 (Brígido, C.; Glick, B.R.; Oliveira, S. Survey of Plant Growth-Promoting Mechanisms in Native Portuguese Chickpea Mesorhizobium Isolates. Microbial Ecology 2017, 73, 900-915, doi:10.1007/s00248-016-0891-9.)).

### Composition of the minimal medium (MM):

MgSO₄.7H₂0 = 150 mg/L,
CaCl₂.2H₂O = 50 mg/L,
FeCl₃ = 6 mg/L,
NaCl = 50 mg/L,
KH₂PO₄ = 300 mg/L,
Glutamic acid = 1.1 g/L,
Sucrose = 2.5 g/L,
Biotin = 0.2 mg/L,
Thiamine-HCl = 0.1 mg/L, and
Calcium pantothenate == 0.1 mg/L,

The Minimal Medium was described by Nascimento *et al.* 2012 (Nascimento, F.X.; Brígido, C.; Glick, B.R.; Oliveira, S.; Alho, L. Lett Appl Microbiol 2012, 55, 15-21, doi:10.1111/j.1472-765X.2012.03251.x) .

The results (Table 3) show that strains **T1** and **T2** are highly tolerant to manganese (≥ 1 mM), while strain **M1** is moderately tolerant (0.1-0.5 mM). However, strain **M2** was only able to grow in a complex 1/10 medium (liquid MLA diluted 10 times in sterile distilled water), being able to tolerate 0.5 mM MnSO₄ and also managed to improve SE, when subject to manganese stress in plant tests. Thus, all strains are able to grow at a 0.5 mM (30 ppm) concentration which corresponds to the levels found in the soil and characterized as toxic levels of Mn.

**Table 3. Saprophytic tolerance of strains to different concentrations of manganese**

| | 0mM | 0.1mM | 0.25mM | 0.5mM | 1mM | 2.5mM | 5mM |
|---|---|---|---|---|---|---|---|
| T1 | + | + | + | + | + | + | d |
| T2 | + | + | + | + | + | + | d |
| M1 | + | + | + | d | - | - | - |
| M2 | + | + | + | + | - | - | - |

| | | | | | | | |
|---|---|---|---|---|---|---|---|
| +, grows; d, weak growth; - does not grow | | | | | | | |

### Example 6. Ability to produce IAA by microbial strains

Each of the four microbial strains **T1, T2, M1** and **M2** was inoculated in 4 mL of minimal liquid medium M9 supplemented with 250 µg/mL tryptophan and then incubated at 30°C until reaching the stationary growth phase (48-72 H). To 1 mL of the supernatant of these cultures 4 mL Salkowski's reagent was added, followed by an incubation for 20-30 min at room temperature (∼ 25°C). After this incubation, the absorbance of each **mixture** (supernatant + Salkowski's reagent) was measured at 535 nm, the concentration of IAA was determined by comparison with a standard curve previously obtained with known concentrations of IAA ranging from 0.01 to 100 µg/mL. The results are shown in table 4.

Within the scope of the present invention, "IAA-like molecules" are those that retain the indole ring and a similar structure, such as 3-indolpyruvic acid, indol-butyric acid, among others.

**Table 4. Microbial production of IAA and IAA-like molecules**

| **Strain** | **IAA and IAA-like molecules (µg/mL)** |
|---|---|
| **T1** | 32.3 |
| **T2** | 30.6 |
| **M1** | 6.9 |
| **M2** | 4.8 |

The high concentrations of IAA and IAA-like molecules (above 50 pg/mL) are harmful to the development and growth of plants, since they disturb the balance between auxins and other phytohormones.

Strains T1 and T2 produced a greater relative concentration of auxins, about 5 times more than strains M1 and M2. The levels of IAA and IAA-like molecules produced by strains M1 and M2 are relatively low, but those levels of IAA and IAA-like produced by strains T1 and T2 are moderate and suitable for promoting plant growth (Duca, D., Lorv, J., Patten, CL, Rose, D., & Glick, BR (2014). Indole-3-acetic acid in plant-microbe interactions. Antonie Van Leeuwenhoek, 106(1), 85-125.doi: 10.1007/s10482-013-0095-y).

In this way, it is demonstrated that the inoculation of the microbial strains of the present invention is advantageous for the growth of these plants, considering their ability to produce auxins.

### Example 7. Compositions of microbial strains

The strains isolated and tested, according to Examples 1 to 7, were used for the production of microbial compositions based on weighted criteria, which favor the complementarity of characteristics, namely the nitrogen fixing ability and the tolerance to high Mn concentrations, presented by each strain.

The tested microbial compositions comprise the two strains of *R. leguminosarum sv trifolii* **(T1 and T2)** and the two strains of *E. medicae* **(M1 and M2).** The final composition contains a quantity of bacteria from each strain varying between 30 and 70%. The microbial strains were in suspension, in buffered saline solution, with the number of microbial cells varying from 10⁶ to 10⁸ per mL. Table 5 presents some of the preferred Base Microbial Compositions (BMC).

**Table 5. Base Microbial Compositions**

| **Composition ID** | **STRAINS** | | | |
|---|---|---|---|---|
| | **% T1** | **% T2** | **% M1** | **% M2** |
| **BMC-01** | 25.0 | 25.0 | 25.0 | 25.0 |
| **BMC-02** | 45.0 | 5.0 | 45.0 | 5.0 |
| **BMC-03** | 5.0 | 45.0 | 5.0 | 45.0 |
| **BMC-04** | 37.5 | 12.5 | 37.5 | 12.5 |
| **BMC-05** | 12.5 | 37.5 | 12.5 | 37.5 |
| **BMC-06** | 40.0 | 40.0 | 10.0 | 10.0 |

### Example 8. Effect of BMCs on promoting plant growth

The effect of the microbial compositions of the invention, as described in the previous Example, in promoting the development of forage leguminous plants was measured by inoculating subterranean clover (*T. subterraneum*) and lucerne (*M*. *polymorpha*) seeds.

A composition was obtained, as described in the previous Example, containing the 4 microbial strains of the invention, **T1, T2, M1** and **M2,** in a ratio of 1:1:1:1 of each of the strains. This composition (BMC-01), was inoculated, as previously described, in pre-germinated clover and lucerne seeds.

For the cultivation of plants, 7x7x8 cm pots containing a mixture of 1:1 vermiculite-sand were used. As controls, pots with uninoculated plants with and without the addition of nitrogen were used. The pots were placed in plant growth chambers with cycles of 16/8 hours and with temperatures of 24/18°C (day/night) and 65% relative humidity.

After 6 weeks of culture, the plants were harvested. Nodules were counted and aerial parts were dried to determine dry weight. Table 6 presents the results of the effect of inoculation of lucerne and clovers with the composition BMC-01, with and without manganese stress.

**Table 6. Lucerne and clover growth**

| **PLANT** | | **WITHOUT stress (-Mn)** | **WITH stress (+Mn)** |
|---|---|---|---|
| **Total SE (%)** | | 93.38 | 104.05 |
| ***Medicago*** | **SE (%)** | 115.15 | 128.08 |
| | **NN** | 84.00 | 87.00 |
| ***Trifolium*** | **SE (%)** | 81.58 | 92.32 |
| | **NN** | 105.00 | 179.00 |

| | | | |
|---|---|---|---|
| SE: symbiotic efficiency; Total SE: symbiotic efficiency resulting from the sum of clover and lucerne dry weights of each pot. NN: number of nodules | | | |

### Example 9. Effect of BMCs on soils with Manganese toxicity

The effect of the microbial compositions of the invention on soils showing manganese-associated toxicity was assessed. For this purpose, tests were carried out on "Montado" soils, therefore acidic soils with manganese toxicity.

Not only were the host plants of the strains of the invention used, i.e. clovers and lucerne, but also a species of grass in association with these legumes. Thus, the effect on different plants was assessed, on the one hand the legumes and on the other the *Lolium multiflorum* (grass).

Three seeds of each of the plants *T. subterraneum, T. resupinatum, M. polymorpha* and *Lolium multiflorum* were placed in pots with a 22 cm diameter and 22 cm high, containing soil that presents Manganese toxicity. These seeds were inoculated with the BMC-01 composition of the invention, containing 10⁸ cfu/mL of each of the 4 strains suspended in isotonic saline solution.

Two control treatments were carried out (without inoculation), wherein one had no nitrogen fertilization (negative control), and the other had nitrogen fertilization, of 70 ppm N in the form of NH₄NO₃ applied at sowing (positive control).

The tests were carried out in an climatized greenhouse with a maximum temperature of 30°C, with the results being shown in table 7.

Vegetable production was measured using the dry weight of the aerial parts of the plants. The plants were harvested and dried in a ventilation oven at 55°C for 3 to 5 days.

The nitrogen content of the aerial parts was determined after drying the plants. The plants were ground and analyzed by determining the amount of nitrogen in a "LECO CN analyzer".

**Table 7. Effect of BMCs in "Montado" soil with natural manganese toxicity**

| | **Shoot dry weight (g/pot)** | **Nitrogen content (mg/pot)** |
|---|---|---|
| **Nitrogen-free control** | 1,064 a | 8.787 a |
| **Nitrogen control** | 1,620 b | 23.703 b |
| **Inoculation of CMB-01** | 2,464 c | 14,100 c |

Different letters in the column indicate significant differences (p≤0.05), according to the analysis of factorial variance with separation of LSD means.

These results show that inoculation with the CMB-01 composition of the invention increased by more than twice the plant or biomass production measured in dry weight of the aerial parts of the plants, compared to control plants with no nitrogen, and one and a half times compared to control plants with nitrogen.

However, if nitrogen availability is not a limiting factor, it is possible that beneficial effects on plants may exist due to the presence of other mechanisms for promoting plant growth in these strains other than atmospheric N₂ fixation, such as IAA synthesis.

### Example 10. Effect of BMCs with different amounts of T1/T2 and M1/M2

Comparative tests were carried out with inoculation of clover and lucerne plants with microbial compositions containing different amounts and ratios of each strain T1, T2, M1 and M2, namely composed of the **6 BMCs in Table 5,** presented in Example 7, and also for 4 compositions containing 100% of each strain of **T1 (CMB-T1), T2 (CMB-T2), M1 (BMC-M1)** or **M2 (BMC-M2).**

The inoculation conditions of BMCs and plant growth, as well as their assessment, were identical to those described in the previous Example, i.e. tests were carried out in "Montado" soils, therefore acidic soils with manganese toxicity.

The results show that inoculation with the BMC-01 composition (25% of each strain T1, T2, M1, M2) favored the growth of the plants, compared to the remainder compositions.

## Claims

1. A microbial composition, selected from a group of strains of *Ensifer medicae* characterized for consisting of strains M1 and M2, individually or in combination, wherein:
- **M1** is *Ensifer medicae,* identified as **RMP15** and with accession number **CECT 9931,** and
- **M2** is *Ensifer medicae,* identified as **MMP28** and with accession number **CECT 9932.**

2. A microbial composition according to claim 1, **characterized by** further comprising at least one strain of *Rhizobium leguminosarum,* selected from:
- **T1** is *Rhizobium leguminosarum,* identified as **TTR4** and with accession number **CECT 9929,**
- **T2** is *Rhizobium leguminosarum,* identified as **RTR25** and with accession number **CECT 9930.**

3. A microbial composition according to claim 2, **characterized by** comprising M1 and/or M2 of *Ensifer medicae* and/or T1 and/or T2 of *Rhizobium leguminosarum* in variable amounts, wherein the *Rhizobium:Ensifer* ratio varies from 50:50%, 25/75%, 75:25%, 30:70%, 70:30%, 40:60%, 60:40%.

4. A microbial composition, according to claim 3, **characterized by** the amount of each of the four strains, in the total of the composition, is variable and according to that shown below:
| **% T1** | **% T2** | **% M1** | **% M2** |
|---|---|---|---|
| 25.0 | 25.0 | 25.0 | 25.0 |
| 45.0 | 5.0 | 45.0 | 5.0 |
| 5.0 | 45.0 | 5.0 | 45.0 |
| 37.5 | 12.5 | 37.5 | 12.5 |
| 12.5 | 37.5 | 12.5 | 37.5 |
| 40.0 | 40.0 | 10.0 | 10.0 |

5. A microbial composition according to any of claims 1 to 4, **characterized by** being in the form of a suspension and by comprising a microbial load greater than about 10³ cfu/mL, preferably from about 10⁶ to 10¹⁶ cfu/mL, more preferably from about 10⁸ to 10¹⁴ cfu/mL, preferably from about 10¹⁰ to 10¹² cfu/mL of the total composition.

6. A process for obtaining a microbial composition, as described in any one of claims 1 to 5, **characterized by** one or more microbial isolates (T1, T2, M1, M2), as described in claim 2, are suspended in a buffered saline solution suitable for the purpose, containing an amount of microbial cells greater than about 10³ cfu/mL, preferably ranging from about 10⁶ to 10¹⁶ cfu/mL, more preferably from about 10⁸ to 10¹⁴ cfu/mL, preferably from about 10¹⁰ to 10¹² cfu/mL of the total saline solution composition.

7. A microbial formulation, **characterized by** comprising a microbial composition, as described in any one of claims 1 to 5, and pharmaceutically acceptable vehicle for agriculture, dispersants, stabilizing agents, pH correction agents, vitamins, growth factor and/or support agents.

8. A microbial formulation according to claim 7, **characterized by** comprising black peat, white peat, alginate, sand, seeds, irrigation solutions, corrective solutions, such as saline solutions and/or solutions containing P or K, Ca, CaO.

9. A method for treating or improving pastures and forages, **characterized by** applying a microbial composition as described in claims 1 to 5, or a microbial formulation as described in claims 7 and 8, to leguminous plants individually or in association with another type of plants.

10. A method according to claim 9, **characterized by** the application of at least one microbial composition to the seeds of those plants, at pre-sowing or sowing time and/or by applying a microbial composition or formulation to the soil where those plants are found, after the seed emerges.

11. A method according to any one of claims 9 or 10, **characterized by** the application of at least one microbial composition, or at least one microbial formulation, is to be repeated after seed germination.

12. A method according to any one of claims 9 to 11 **characterized by** being applied to seeds, seedlings and/or cultures comprising legumes, preferably forage legumes, such as lupins and yellow lupins (*Lupinus* sp. including *L. albus, L luteus, L. angustifolius*), vetches (*Vicia* sp., including *V. sativa,* V. *faba, V. villosa, V. ervilia, V. articulata, V. narboensis, V. benghalensis, V. pannonica*) and sawdust (*Ornithopus sativus*), even more preferably to selected clovers (*Trifolium incarnatum, T. suaveolens, T. michelianum, T. resupinatum, T. vesiculosum, T. subterraneum* ssp *brachycalicinum, T. subterraneum* ssp *yanninicum, T. subterraneum ssp subterraneum, T. pratense, T campestre, T. tomentosum, T. angustifolium, T. fragiferum*) and lucernes (*Medicago polymorpha, M. truncatula, M. arabica, M. scutellata, M. murex),* individually or in association with other plants, preferably other forage plants, more preferably, forage grasses.
